**Europäisches Patentamt**

**European Patent Office**

**Office européen des brevets**

(19)

(11) Veröffentlichungsnummer: **0 199 375**
**B1**

(12) # EUROPÄISCHE PATENTSCHRIFT

(45) Veröffentlichungstag der Patentschrift:
**29.11.89**

(51) Int. Cl.⁴: **G 01 N 29/00, A 61 B 8/00**

(21) Anmeldenummer: **86200242.5**

(22) Anmeldetag: **19.02.86**

(54) **Verfahren zur Bestimmung einer Schallbrechungsindexverteilung in einem Untersuchungsbereich und Anordnung zur Durchführung des Verfahrens.**

(30) Priorität: **20.02.85 DE 3505764**

(43) Veröffentlichungstag der Anmeldung:
**29.10.86 Patentblatt 86/44**

(45) Bekanntmachung des Hinweises auf die Patenterteilung:
**29.11.89 Patentblatt 89/48**

(84) Benannte Vertragsstaaten:
**BE DE FR GB**

(56) Entgegenhaltungen:
**DE-A- 2 827 423**
**US-A- 4 279 157**

(73) Patentinhaber: **Philips Patentverwaltung GmbH,**
**Wendenstrasse 35 Postfach 10 51 49,**
**D-2000 Hamburg 1 (DE)**

(84) Benannte Vertragsstaaten: **DE**

(73) Patentinhaber: **N.V. Philips' Gloeilampenfabrieken,**
**Groenewoudseweg 1, NL-5621 BA Eindhoven (NL)**

(84) Benannte Vertragsstaaten: **BE FR GB**

(72) Erfinder: **McKinnon, Graeme-Colin, Dr.,**
**Skandinavienallee 2, D-2086 Ellerau (DE)**

(74) Vertreter: **Hartmann, Heinrich, Dipl.-Ing. et al, Philips Patentverwaltung GmbH**
**Wendenstrasse 35 Postfach 10 51 49,**
**D-2000 Hamburg 1 (DE)**

**Beschreibung**

Die Erfindung betrifft ein Verfahren zur Ermittlung der inneren Struktur eines Körpers, wobei eine Vielzahl von Ultraschallstrahlen von je einem oder mehreren Sendeelementen einer Ultraschallsendeanordnung den Körper in unterschiedlichen Richtungen durchsetzt und von einer oder mehreren Empfangselementen einer Ultraschallempfängeranordnung in elektrische Signale umgesetzt wird, wobei die Laufzeiten der Ultraschallstrahlen gemessen werden und wobei aus den Laufzeiten und einer vorgewählten, der Struktur des Körpers angenäherten Verteilung der Schallbrechungsindizes wenigstens einmal für jeden Schallstrahl sein Weg zur Errechnung seiner Laufzeit zumindest annähernd bestimmt wird und aus gemessener und errechneter Laufzeit der Ultraschallstrahlen Korrekturdaten zur schrittweisen Korrektur der Schallbrechungsindexverteilung erhalten werden. Die Erfindung betrifft weiter eine Anordnung zur Durchführung dieses Verfahrens.

Ein solches Verfahren und eine solche Anordnung sind aus der US-PS 4 279 157 bekannt. Das Rechenverfahren ist darüber hinaus noch im Detail beschrieben in H. Schromberg, «Nonlinear image reconstruction from projections of ultrasonic travel times and electric current densities», Mathematical aspects of computerized tomography (proceeding, Oberwolfach 1989, editors G. T. Herman and F. Natterer), Springer-Verlag, S. 270–291. Der Vorteil des bekannten Verfahrens besteht darin, daß die Berechnung der Schallbrechungsindexverteilung nicht auf der Annahme beruht, der Schall würde sich auf geradlinigen Wegen im Körper ausbreiten. Aufgrund der Tatsache nämlich, daß in einem realen Körper der Schallbrechungsindex sich mit dem Ort ändert, verläuft der Weg des Schalls durch den Körper nicht geradlinig, so daß eine Schallbrechungsindexverteilung, deren Ermittlung auf der oben erwähnten Annahme beruht, notwendigerweise falsch ist.

Bei dem bekannten Verfahren wird der in der Regel nicht geradlinige Weg des Ultraschallstrahles durch den Untersuchungsbereich bzw. den untersuchten Körper mittels eines Satzes miteinander gekoppelter Differentialgleichungen erster Ordnung unter der Randbedingung gelöst, daß der Schallstrahl vom Schallsendeort ausgehen und am Schallempfangsort enden muß. Dabei wird vom Sendeort ausgehend zunächst eine Anfangsrichtung vorgegeben, bis der nächste Bildpunkt erreicht ist. Für diesen nächsten Bildpunkt wird dann aufgrund der Differentialgleichung eine neue Richtung bis zum übernächsten Bildpunkt errechnet usw., so daß sich ein Weg ergibt, dessen Richtung sich schrittweise verändert. In der Regel erreicht dieser Weg jedoch nicht den Ultraschallempfangsort, sondern geht an ihm vorbei. Aus dem Abstand des Ultraschallempfangsortes von dem Ultraschallpfad läßt sich eine Korrektur für die Anfangsrichtung des Ultraschallstrahls ableiten, wonach das Verfahren erneut durchgeführt wird. Diese Rechenzyklen werden so oft durchlaufen, bis der Ultraschallempfangsort wenigstens annähernd erreicht ist. Es leuchtet ein, daß dieses Rechenverfahren sehr aufwendig ist.

Aufgabe der Erfindung ist es, zur Bestimmung des Weges des Ultraschallstrahles durch den Untersuchungsbereich ein Verfahren anzugeben, bei dem der ermittelte Weg stets den Ultraschallsendeort mit dem Ultraschallempfangsort verbindet. Diese Aufgabe wird erfindungsgemäß dadurch gelöst, daß zur Bestimmung des Weges eines Ultraschallstrahles die Integralgleichungen

$$Y = \int_{x0}^{x} \frac{w}{n} \int_{x0}^{s} gw \, dt \, ds + A \int_{x0}^{xH} \frac{w}{n} \, ds \qquad (1)$$

und

$$Z = \int_{x0}^{x} \frac{w}{n} \int_{x0}^{s} hw \, dt \, ds + B \int_{x0}^{x} \frac{w}{n} \, ds \qquad (2)$$

gelöst werden, wobei x bzw. t, s die Richtung der Verbindungsgeraden zwischen dem Schallsendeort x0 und dem Schallempfangsort xf bezeichnen, Y und Z den Abstand des Weges von dieser Geraden in zwei zueinander senkrechten Richtungen bezeichnen, n den Schallbrechungsindex und g bzw. h dessen Gradienten in y- bzw. z-Richtung und w den Differentialquotienten des Weges nach x darstellen und A und B jeweils derart gewählte Faktoren sind, daß die Bedingungen $Y(xf) = 0$ und $Z(xf) = 0$ erfüllt sind.

Bei diesen Gleichungen sind – für jeden Weg gesondert – die Faktoren A und B so gewählt, daß Y und Z an der Stelle xf Null sind, was gleichbedeutend damit ist, daß der ermittelte Weg am Ultraschallempfangsort endet. Dies ist immer dann der Fall, wenn für A bzw. B die Beziehungen

$$A = - \frac{\displaystyle\int_{x0}^{xf} \frac{w}{n} \int_{x0}^{s} gw \, dt \, ds}{\displaystyle\int_{x0}^{xf} \frac{w}{n} \, ds} \qquad (3)$$

$$B = - \frac{\displaystyle\int_{x0}^{xf} \frac{w}{n} \int_{x0}^{xf} hw \, dt \, ds}{\displaystyle\int_{x0}^{xf} \frac{w}{n} \, ds} \qquad (4)$$

gelten. Diese Gleichungen sind, wie noch gezeigt wird, mittels eines Digitalrechners sehr leicht zu lösen.

Es läßt sich zeigen, daß das Integral über dem Brechungsindex auf dem so ermittelten Weg praktisch ein Minimum darstellt im Hinblick auf die Integrale über die sonst noch zwischen den beiden Punkten denkbaren Wege. Der so berechnete Weg entspricht daher zumindest in guter Näherung demjenigen Weg, den ein Ultraschallstrahl durch einen Untersuchungsbereich nehmen würde, in dem die zugrundeliegende Brechnungsindexverteilung vorgegeben ist (Fermat'sches Prinzip). Daß der berechnete Weg nicht exakt mit dem tatsächlichen Weg übereinstimmt, liegt daran, daß die ortsabhängigen Größen n, g, h, w, über die gemäß Gleichungen (1) und (2) jeweils integriert wird, für die berechneten Punkte Y und Z zunächst noch gar nicht bekannt sind. Erst wenn der Weg bekannt ist, kann man die Größe dieser Parameter auf dem Weg bestimmen und davon ausgehend einen neuen Weg berechnen. Demgemäß sieht eine Ausgestaltung der Erfindung vor, daß zur iterativen Bestimmung des Weges eines Schallstrahles die Werte n, g und h jeweils für die Koordinaten des in einem vorhergehenden Zyklus ermittelten Weges bestimmt werden.

Es ist möglich, daß der tatsächliche Weg des Ultraschallstrahles wenigstens auf Teilen seiner Länge zwischen einem ersten errechneten Weg und einem zweiten errechneten Weg liegt, bei dessen Berechnung vom ersten Weg ausgegangen wird. Das bedeutet, daß für die betreffenden Teile des Weges die Koordinaten des ersten Weges zu klein (oder zu groß) und die Koordinaten des zweiten Weges zu groß (oder zu klein) waren. Dieses «Überschwingen» bzw. «Oszillieren» der gefundenen Lösungen läßt sich nach einer Weiterbildung der Erfindung dadurch vermeiden, daß aus den neu berechneten Koordinaten Y, Z und den bisher für den gleichen x-Wert geltenden Koordinaten Ya, Za die Koordinaten Yn, Zn des neuen Weges nach Gleichungen

$$Y_n = dY_a + (d-1)Y \qquad (5)$$

$$Z_n = dZ_a + (d-1)Z \qquad (6)$$

berechnet werden, wobei d ein vorgegebener Faktor ist, der der Bedingung $0 < 1 < 1$ genügt.

Zur Durchführung des Verfahrens ist eine Anordnung vorgesehen mit einer aus einer Mehrzahl von Sendeelementen bestehenden Ultraschallsendeanordnung und einer separaten, aus einer Mehrzahl von Empfängerelementen bestehenden Ultraschallempfängeranordnung, mit Mitteln zum Selektieren von Sende- und Empfangselementen derart, daß die jeweils aktivierten Sende- und Empfangselemente auf parallelen Verbindungsgeraden liegen, mit Mitteln zum Messen der Laufzeit der Schallstrahlen zwischen dem jeweils aktivierten Sender- und Empfängerelementen und mit einer Rechenanordnung zur Bestimmung der Brechungsindexverteilung in einem zwischen der Ultraschallsendeanordnung und der Ultraschallempfangsanordnung befindlichen Untersuchungsbereich. Dabei ist vorgesehen, daß die Rechenanordnung Mittel zur Berechnung des Abstandes des Ultraschallstrahles von der zugehörigen Verbindungsgeraden an einer Vielzahl von Punkten nach einer Integralgleichung aufweist.

Die Erfindung wird nachstehend anhand der Zeichnung näher erläutert. Es zeigen

Fig. 1 eine schematisch dargestellte Ultraschallsende- und empfangsanordnung und den dazwischenliegenden Unterschungsbereich,

Fig. 2 die Aufeinanderfolge der Rechenschritte zur Bestimmung der y-Koordinaten des Weges des Ultraschallstrahles,

Fig. 3 bis 6 die Flußdiagramme verschiedener Unterprogramme zur Durchführung einzelner Rechenschritte nach Fig. 2, und

Fig. 7 eine Anordnung zur Durchführung des erfindungsgemäßen Verfahrens in schematischer Darstellung.

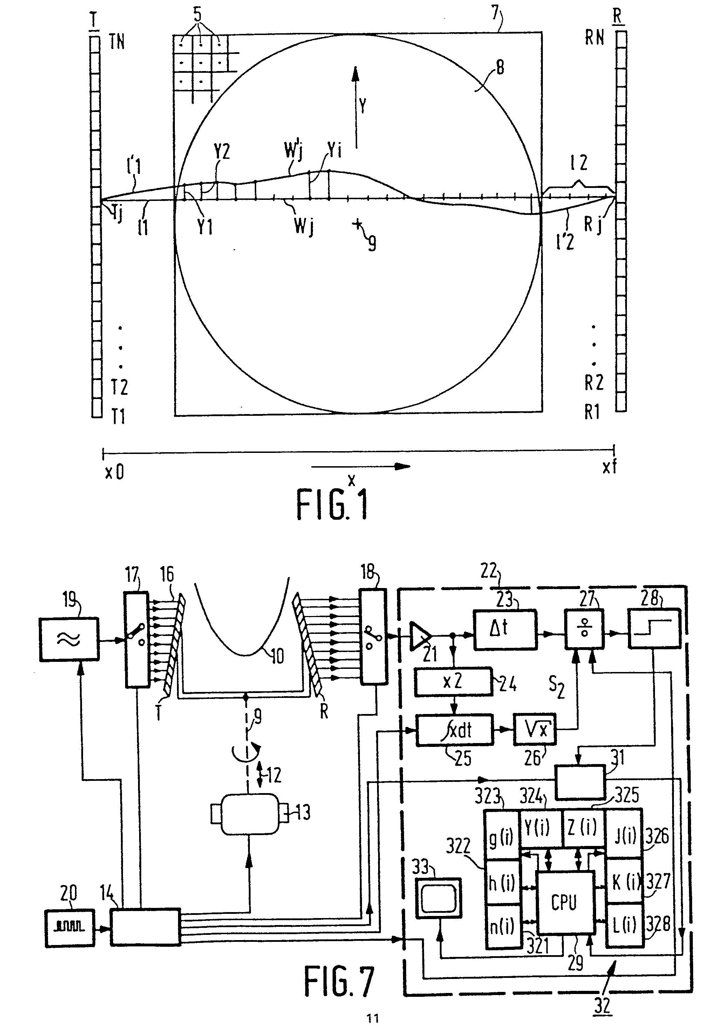

In Fig. 1 ist mit T eine Ultraschallsendeanordnung und R eine Ultraschallempfangsanordnung bezeichnet. Die Ultraschallsendeanordnung besteht aus einer Zeile von N Sendeelementen T1...TN. Ebenso besteht die Ultraschallempfangsanordnung R aus N Empfangselementen R1...RN. Ein geeigneter Wert für N ist N=64. Mit noch mehr Sende- und Empfangselementen läßt sich zwar eine bessere Auflösung erreichen oder ein größerer Untersuchungsbereich erfassen, jedoch steigt damit auch der Aufwand. Der Abstand der beiden parallel zueinander verlaufenden Zeilen T und R ist größer als deren Länge. Sie befinden sich in einem nicht näher dargestellten, mit Wasser oder einer anderen geeigneten Flüssigkeit gefüllten Tank und sind mittels eines nicht näher dargestellten Motorantriebes um die vertikal zur Zeichenebene verlaufende Drehachse 9 drehbar. Der zwischen den Zeilen T und R liegende Untersuchungsbereich, innerhalb dessen die Schallbrechungsindexverteilung ermittelt werden kann, ist durch den Kreiszylinder 8 begrenzt, dessen Achse mit der Rotationsachse 9 zusammenfällt.

Durch eine nicht näher dargestellte Steuerelektronik werden die Sende- und Empfangselemente so geschaltet, daß immer das Empfangselement auf Empfang geschaltet ist, das zusammen mit dem gerade sendenden Sendeelement auf einer zur x-Richtung parallelen Verbindungsgeraden liegt, so daß z. B. die Elemente T1, R1; T2, R2; ... Tj, Rj zusammen eingeschaltet sind. Die dabei z. B. vom Schallelement Tj erzeugte Ultraschallenergie erreicht das zugeordnete Empfangselement Rj auf einem Weg W'j, der von der Verbindungsgeraden Wj zwischen den beiden Elementen in der Regel abweicht. Für jeden dieser Ultraschallwege wird die Laufzeit gemessen, und wenn dies für sämtliche N Paare durchgeführt ist, wird die Ultraschallwandleranordnung T, R um einen Winkel z.B. 360°/N gedreht, wonach der gleiche Meßzyklus wiederholt wird. Die Drehung und der anschließende Meßzyklus werden insgesamt N mal durchgeführt. Diese Messungen werden für andere Schichten wiederholt, wobei die Ultraschallwandleranordnungen T und R relativ zum Untersuchungsbereich in z-Richtung, d.h. senkrecht zur Zeichenebene verschoben werden.

Anschließend erfolgt die Rekonstruktion der Schallbrechungsindexverteilung innerhalb des Untersuchungsbereiches 8. Dazu werden aufgrund einer vorgegebenen, der erwarteten Brechungsindexverteilung angenäherten Verteilung für jedes Paar (z.B. Tj, Rj) jede der erwähnten Richtungen und jede Schicht die Wege W'j bestimmt. Aus dem Vergleich der errechneten Laufzeit, die das Wegintegral des Brechungsindex ist, mit der gemessenen Laufzeit wird ein Korrekturwert errechnet und die Schallbrechungsindizes entlang des errechneten Weges W'j und innerhalb des Zylinders 8 werden so korrigiert, daß die errechnete Laufzeit mit der gemessenen Laufzeit übereinstimmt. Dies wird für jedes der Paare T1, R1...TN, RN wiederholt, für jede Richtung und für jede Schicht. Dabei ergibt sich eine Brechungsindexverteilung, die der tatsächlichen Brechungsindexverteilung schon besser entspricht wie die vorherige und die ihrerseits noch durch weitere Iterationszyklen verbessert werden kann.

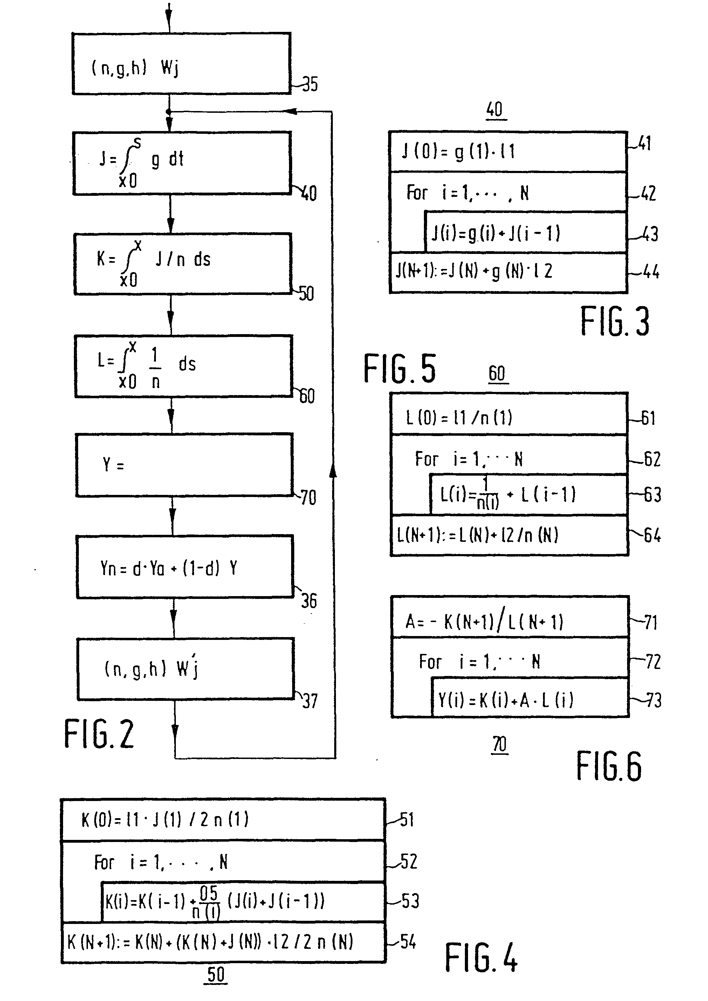

Insoweit als bisher beschrieben ist das Verfahren im wesentlichen aus den eingangs erwähnten Veröffentlichungen bekannt, auf die wegen der Einzelheiten Bezug genommen wird. Die Erfindung unterscheidet sich von dem bekannten Verfahren durch die Ermittlung der Wege W'j für die Ultraschallstrahlen, die den aufwendigsten Teil des gesamten Iterationsverfahrens darstellt. Dieses Verfahren wird anhand der Fig. 2 nachfolgend näher erläutert.

Ausgegangen wird (Block 35) von einer Näherungsverteilung der Parameter n, g, h. Dabei ist n der Schallbrechungsindex an dem jeweiligen Ort und für g und h gilt

$$g = \frac{\delta n}{\delta y} \qquad (7)$$

$$h = \frac{\delta n}{\delta z} \qquad (8)$$

An sich muß auch noch der Wert w vorhanden sein, der der Gleichung

$$w = \left( 1 + \left(\frac{dy}{dx}\right)^2 + \left(\frac{dz}{dx}\right)^2 \right)^{\frac{1}{2}} \qquad (9)$$

genügt, doch wird dieser für den gesamten Bereich gleich 1 gesetzt. Das erleichtert die Berechnung wesentlich und verursacht nur einen geringen Fehler, weil w in den für die Praxis wichtigen Fällen, beispielsweise bei der Mammauntersuchung, in der Nähe von 1 liegt.

Da der Ultraschall sich in dem Untersuchungsbereich nicht geradlinig ausbreitet, genügt es nicht, diese Parameter für einen ebenen Bereich zu kennen; sie müssen vielmehr für einen dreidimensionalen Bereich bekannt sein. Es muß daher ein Speicher vorhanden sein, in dem für jedes Volumenelement – im folgenden kurz voxel genannt – die Parameter n, g, h gespeichert sind. Alle voxel zusammen bilden einen kubusförmigen Raum 7, dessen Querschnitt in der Zeichenebene der Fig. 1 ein Quadrat mit einer dem Durchmesser des Zylinders 8 entsprechenden Kantenlänge ist, das aus N × N voxeln besteht, von denen einige in Fig. 1 mit 5 bezeichnet sind.

Die Näherungsverteilung von n, g und h, von der bei der Rekonstruktion ausgegangen wird, kann an sich beliebig sein; je besser die Anfangsverteilung jedoch mit der tatsächlichen Brechungsindexverteilung übereinstimmt, desto geringer ist der Rechenaufwand. Eine Brechungsindexverteilung, bei der für alle voxel die gleichen Werte n sowie g = 0 und h = 0 gespeichert sind, wäre daher verhältnismäßig ungünstig. Eine bessere Näherungsverteilung ergibt sich, wenn man zunächst unterstellt, daß sich der Ultraschall geradlinig ausbreitet und aus der gemessenen Laufzeit, die dem Wegintegral des Brechungsindex entspricht, in gleicher Weise die Brechungsindexverteilung rekonstruiert, wie man bei der Computertomographie aus den gemessenen Absorptionswerten, die dem Wegintegral über die Absorptionskoeffizienten entsprechen, die Verteilung der Absorptionskoeffizienten ermittelt. Aus diesen Brechungsindizes können die Differentialquotienten g und h als Differenzenquotient berechnet werden.

Im nächsten Schritt (Block 40) wird das Integral J ermittelt, das dem inneren Integral in dem Doppelintegral nach Gleichung (1) entspricht, wenn w = 1 gesetzt wird.

Mit Hilfe der so ermittelten Werte J wird das Integral K ermittelt, das dem Doppelintegral der Gleichung (1) entspricht (Block 50), wobei wieder w = 1 gesetzt ist. Schließlich wird noch das Integral L berechnet (Block 60), das dem zweiten Integral in Gleichung (1) für w = 1 entspricht. Mit Hilfe der so errechneten Werte J, K und L lassen sich die y-Koordinaten entsprechend Gleichung (1) ausrechnen (Block 70). In analoger Weise werden die Z-Werte berechnet. Dazu muß lediglich in Block 40 der Parameter g durch h ersetzt werden.

Grundsätzlich können die so gefundenen Koordinaten Y und Z als Koordinaten des neuen Weges betrachtet werden. Es zeigt sich jedoch, daß diese Koordinaten in der Regel jenseits des tatsächlichen Weges liegen, wenn man von dem bisherigen Weg Wj ausgeht. Dieses «Überschwingen» bzw. «Oszillieren» bei der Berechnung des Weges des Ultraschallstrahles durch den Untersuchungsbereich läßt sich aber vermeiden bzw. verringern, wenn man die Koordinaten für den neuen Weg nach der Gleichung (5) berechnet. Dabei ist Yn der Wert der y-Koordinate für den neuen Weg, Y der zuvor (Block 70) berechnete Y-Wert und Ya die y-Koordinate des bisherigen Weges Wj an der gleichen Stelle x, und d ist ein Dämpfungsfaktor, der kleiner ist als 1 und größer als 0. Je kleiner dieser Wert ist, desto stärker ist das «Überschwingen» bzw. «Oszillieren»; je dichter dieser Wert bei 1 liegt, desto stärker wird dieses «Überschwingen» bzw. «Oszillieren» unterdrückt, desto öfter muß man jedoch auch den Weg iterativ berechnen, damit der durch Rechnung gefundene Weg dem tatsächlichen Weg auch entspricht. Analog dazu werden die z-Koordinaten für den neuen Weg nach der Gleichung (6) bestimmt. In der Regel entspricht der berechnete Weg nicht exakt dem Weg, den ein Ultraschallstrahl bei der vorgegebenen Brechungsindexverteilung nehmen würde; dies nicht nur wegen der Manipulation in Block 36, sondern auch deshalb, weil die Gleichungen (1) und (2) nur dann exakt die Koordinaten des tatsächlichen Weges bezeichnen, wenn die Parameter g, h, n, w in den Gleichungen (1) und (2) jeweils den tatsächlichen Parametern auf diesem Weg, d. h. am Ort x, Y, Z entsprechen. Da dieser Weg bzw. die Koordinaten Y und Z a priori aber unbekannt sind, werden die Parameter g, h, n, w in der Regel diesen Bedingungen nicht genügen. Es ist jedoch möglich, den errechneten Weg iterativ dem Weg anzunähern, den ein Ultraschallstrahl bei der angenommenen Brechungsindexverteilung tatsächlich nehmen würde. Zu diesem Zweck werden die Parameter n, g, h auf dem zuvor neu berechneten Weg W'j neu bestimmt. Da der Weg W'j in der Regel nicht exakt durch die Zentren der einzelnen voxel verläuft, müssen die Parameter n, g, h durch Interpolation der für diejenigen voxel gespeicherten Werte ermittelt werden, zwischen deren Zentren dieser Weg verläuft (Block 37). Mit den so gefundenen Werten kann dann der Weg erneut berechnet werden (Blöcke 40, 50, 60, 70, 36 und 37), wobei der dann gefundene Weg in der Regel besser demjenigen Weg entspricht, den ein Schallstrahl bei der vorgegebenen Brechungsindexverteilung nehmen würde, als der davor gefundene Weg.

Das Diagramm der Fig. 2 erläutert die Berechnung des Weges nur für eine einzige Richtung (der Ultraschallwandleranordnung T, R) in bezug auf den Untersuchungsbereich 8 und nur für ein einziges Elementenpaar Tj, Rj. Die Berechnungen der anderen Wege sind jedoch unabhängig von der Berechnung für den Weg Wj, und sie können daher unabhängig davon, also auch zeitlich parallel, durchgeführt werden. Auch die Integralberechnung in den Blöcken 40 und 60 kann voneinander unabhängig durchgeführt werden, und die Teilintegrale, die in Block 40 berechnet werden, können jeweils unmittelbar anschließend schon in Block 50 weiterverarbeitet werden.

Fig. 3 zeigt in einem detaillierteren Flußdiagramm (Nassi-Schneiderman-Diagramm) die Schritte, die bei der Berechnung des Integrals J ausgeführt werden (Block 40). Dabei wird zunächst ein Wert J(o) bestimmt. Dieser Wert wäre 0, wenn die Ultraschallsendeanordnung T mit dem Rand des Bereiches 7 zusammenfiele, für dessen einzelne voxel die Werte n, g und h gespeichert sind. Da sich die Ultraschallsendeanordnung jedoch bei x0 im Abstand l1 von diesem Bereich befindet, wird dieser Anfangswert nach der Gleichung

$$J(o) = g(1)l1 \qquad (10)$$

bestimmt, wobei l1 der Abstand zwischen dem Bereich 7 und der Ultraschallsendeanordnung T ist und g(1) der Wert auf dem ersten voxel ist, das die Verbindungsgerade Wj zwischen dem Sendeelement Tj und dem Empfangselement Rj durchschneidet. Anschließend werden für alle N voxel, die sich auf dem Strahl Wj befinden, die Integralwerte J(i) nach der Gleichung

$$J(i) = g(i) + J(i-1) \qquad (11)$$

berechnet (Schritte 42, 43). Danach ist also der Wert J für das i-te voxel auf dem Wege Wj gleich der Summe aus dem Wert g für dieses voxel und dem Wert J für das vorangegangene voxel (i−1). Ist dies für alle N voxel durchgeführt, wird zu dem zuletzt gefundenen Wert J(N) noch das Produkt g(N)l2 hinzuaddiert, wodurch der Tatsache Rechnung getragen wird, daß sich die Zeile R mit den Empfangselementen im Abstand l2 von dem Bereich 7 befindet (Schritt 44).

Die Werte J(i) für die Werte i=1,...N stellen den numerischen Wert des Integrals an den Orten x(1)...x(N) dar, wobei x() jeweils den x-Wert im Zentrum des voxels () darstellen. Der Wert J(N+1) entspricht diesem Integral am Ort x = xf, d. h. am Ort des Empfängerelementes Rj.

Die so gefundenen Werte werden im Block 50 weiterverarbeitet, dessen Verarbeitungsschritte in Fig. 4 dargestellt sind. Das numerische Integral K(i) für das i-te voxel wird dabei wiederum aus der Summe des numerischen Integrals K(i−1) für den vorangegangenen Punkt (i−1) und dem durch den Brechungsindex n(i) für dieses voxel dividierten arithmetischen Mittelwert der Integrale J(i)+J(i−1) berechnet (Schritt 53). Für den Anfangswert K(o) gilt

$$K(o) = l1 + J(1)/2n(1) \qquad (12)$$

und für den Wert K(N+1) an der Stelle x=xf gilt Gleichung

$$K(N+1) = K(N) + (K(N) + J(N))l2/2n(N)) \qquad (13).$$

Fig. 5 zeigt das Flußdiagramm für den Block 60 mit den Schritten 61, 62, 63 und 64, die sich von den Schritten 41, 42, 43 und 44 lediglich dadurch unterscheiden, daß der Parameter $g(i)$ ersetzt ist durch $1/n(i)$.

Fig. 6 zeigt das Flußdiagramm zur Berechnung der Werte Y aus den Werten K und L. Gemäß Schritt 71 wird zunächst entsprechend Gleichung (3) der Wert A als negativer Quotient der Werte K(N+1) und L(N+1) berechnet. Anschließend werden (Schritte 72 und 73) für $i=1...N$ die zu den Werten $x(i)$ gehörigen Werte $Y(i)$ nach der Beziehung

$$Y(i) = K(i) + AL(i) \qquad (14)$$

berechnet. Die Berechnung der Werte $Z(i)$, die unabhängig von der Berechnung der Wert $Y(i)$, d.h. zeitlich parallel, erfolgen kann, vollzieht sich auf gleiche Weise. Lediglich im Block 40 ist $g(i)$ durch $h(i)$ zu ersetzen.

Wie schon in Verbindung mit Fig. 2 erläutert, erfolgt anschließend gemäß den Gleichungen (5) und (6) die Berechnung von Yn und Zn und danach die Bestimmung der Parameter n, g, h für den neu gefundenen Weg W'j an den Punkten $x(i)$, $Y(i)$ und $Z(i)$, wonach die Blöcke 40...37 erneut durchlaufen werden können. In der Regel sollten zwischen zwei und sechs Iterationen durchgeführt werden. Bei weniger als zwei Iterationen ist die Berechnung zu ungenau und bei mehr als sechs Iterationen ergibt sich praktisch keine Verbesserung mehr.

Es ist sinnvoll, bei der Berechnung der Brechungsindex überall außerhalb des Raumes 7 und in den voxeln am Rande dieses Raumes – also z.B. in dem ersten und dem N-ten voxel auf dem Wege Wj – auf einen konstanten Wert zu setzen, der demjenigen der Flüssigkeit entspricht, die den Untersuchungsbereich umgibt. In diesem Fall können die Rechenschritte 41 und 44 in Fig. 3 entfallen, weil $g(1) = g(N) = 0$ gilt, woraus $J(0) = 0$ und $J(N+1) = J(N)$ folgt.

Bei der Berechnung der Integrale J, K und L, bei denen von den Werten g, k, n längs des neuen Weges W'j ausgegangen wird, ist zu berücksichtigen, daß sich auch l1 bzw. l2 ändern kann – wenn Y(1) oder Z(1) bzw. Y(N) oder Z(N) von Null verschieden ist. In diesem Fall sind an Stelle von l1 bzw. l2 die Werte l'1 und l1'2 (vgl. Fig. 1) in die Blöcke 41, 44, 51, 54 und 61, 64 einzusetzen, die sich nach dem Satz von Pythagoras bestimmen lassen.

In Fig. 7 ist eine Anordnung zur Durchführung des Verfahrens nach der Erfindung dargestellt. Ein nicht näher dargestellter Tank, der ein Untersuchungsobjekt, z.B. eine Mamma 10 aufnimmt, und mit Wasser gefüllt ist, umschließt eine Ultraschallsendeanordnung T und eine Ultraschallempfangsanordnung R, die aus einzelnen zeilenförmig an-geordneten Schallsende- bzw. Schallempfangs-elementen bestehen. Die Ultraschallsendeanordnung ist über den Tank oder einen anderen geeigneten Träger starr mit der Ultraschallempfangsanordnung verbunden und mit dieser zusammen um eine im Zentrum befindliche Drehachse 9 mittels eines Motors 13 drehbar. Die Elemente der Ultraschallsendeanordnung sind über einen Demultiplexer 17 mit einem Impulsgenerator 19 verbunden, und die Elemente der Ultraschallempfangsanordnung R sind über einen Multiplexer 18 mit einer Auswerteeinheit 22 verbunden. Eine an einen Taktgenerator 20 angeschlossene Steuereinheit 14 sorgt dabei dafür, daß immer die zusammengehörigen Sende- und Empfangselemente, z.B. Tj, Rj (vgl. Fig. 1), gleichzeitig aktiviert sind.

Um mit der Anordnung T, R eine andere Schicht des Objektes 10 zu untersuchen, ist diese Anordnung in Richtung des Pfeiles 12, d.h. in Richtung der Drehachse 9, verschiebbar. Diese mechanische Verschiebung kann aber entfallen, wenn die beiden Anordnungen T und R jeweils eine ebene Matrix von Elementen mit mehreren, in Richtung des Pfeiles 12 gegeneinander versetzten Elementen enthalten.

In der Auswerteeinheit 22 wird das Ausgangssignal des Multiplexers 18 durch einen Verstärker 21 verstärkt und über ein Verzögerungsglied 23 einer Dividierschaltung 27 zugeführt. In der Dividierschaltung 27 wird dieses Signal durch ein der Intensität des Ultraschallsignals entsprechendes Signal dividiert, das durch eine an den Ausgang des Verstärkers 21 angeschlossene Quadrierschaltung 24 enthalten ist, die über eine Integrierschaltung 25 und eine Raditierschaltung 26 mit dem Eingang der Dividierschaltung gekoppelt ist, so daß der zeitliche Verlauf des Ausgangssignals der Dividierschaltung 27 von der Intensität der empfangenen Ultraschallsignale weitgehend unabhängig ist. Das Ausgangssignal der Dividierschaltung 27 wird einer Triggerschaltung 28 zugeführt, die einen Zähler 31 stoppt, wenn der Pegel des Ausgangssignals einen vorgegebenen Wert überschreitet. Die Zahl der bis dahin vom Zähler 31 erfaßten Impulse der Steuereinheit 14 ist somit ein Maß für die Laufzeit; jedoch kann die Laufzeit auch so bestimmt werden wie in der DE-OS 3 242 284 beschrieben.

Die gemessenen Laufzeiten werden einer Verarbeitungseinheit zugeführt, die einen Digitalrechner 29 und einen Speicher 32 umfaßt. Von diesem Speicher sind in der Zeichnung nur diejenigen Teile dargestellt, die für die Ermittlung des Weges jeweils von Bedeutung sind. Danach enthält der Speicher Bereiche 321, 322 und 323, in denen die Parameter n, g und h für eine einzige Richtung und alle Wege Wj, deren Verbindungsgeraden in dieser Richtung verlaufen, gespeichert sind. Weiterhin enthält der Speicher Abschnitte 324 und 325, in denen die Koordinaten der daraus berechneten Wege $Y(i)$ und $Z(i)$ gespeichert sind, und schließlich sind Speicherabschnitte 326, 327 und 328 vorgesehen, die die Werte $J(i)$ und $K(i)$, $L(i)$ jeweils nur für einen einzigen Weg aufnehmen. Der Digitalrechner 29 ist so programmiert, daß er die in Verbindung mit den Fig. 1 bis 6 beschriebenen Rechnungen durchführen kann.

**Patentansprüche**

1. Verfahren zur Ermittlung der inneren Struktur eines Körpers, wobei eine Vielzahl von Ultraschallstrahlen von je einem oder mehreren Sendeelementen einer Ultraschallsendeanordnung den Körper in unterschiedlichen Richtungen durchsetzt und von einer oder mehreren Empfangselementen einer Ultraschallempfängeranordnung in elektrische Signale umgesetzt wird,

$$Y = \int_{x0}^{x} \frac{w}{n} \int_{x0}^{s} gw \, dt \, ds + A \int_{x0}^{xH} \frac{w}{n} \, ds \qquad (1)$$

und

$$Z = \int_{x0}^{x} \frac{w}{n} \int_{x0}^{s} hw \, dt \, ds + B \int_{x0}^{x} \frac{w}{n} \, ds \qquad (2)$$

gelöst werden, wobei x bzw. t, s die Richtung der Verbindungsgeraden zwischen dem Schallsendeort x0 und dem Schallempfangsort xf bezeichnen, Y und Z den Abstand des Weges (W'j) von dieser Geraden in zwei zueinander senkrechten Richtungen (y, z)bezeichnen, n den Schallbrechungsindex und g bzw. h dessen Gradienten in y- bzw. z-Richtung und w den Differentialquotienten des Weges (W'j) nach x darstellen und A und B jeweils derart gewählte Faktoren sind, daß die Bedingungen Y (xf) = 0 und Z (xf) = 0 erfüllt sind.

2. Verfahren nach Anspruch 1, dadurch gekennzeichnet, daß zur iterativen Bestimmung des Weges eines Schallstrahles die Werte n, g und h jeweils für die Koordinaten (X, Y, Z) des in einem vorhergehenden Zyklus ermittelten Weges (W'j) bestimmt werden.

3. Verfahren nach einem der Ansprüche 1 oder 2, dadurch gekennzeichnet, daß aus den neu berechneten Koordinaten Y, Z und den bisher für den gleichen x-Wert geltenden Koordinaten Ya, Za die Koordinaten Yn, Zn des neuen Weges nach den Gleichungen

Yn = dYa + (d–1)Y

Zn = dZa + (d–1)Z

wobei die Laufzeiten der Ultraschallstrahlen gemessen werden und wobei aus den Laufzeiten und einer vorgewählten, der Struktur des Körpers angenäherten Verteilung der Schallbrechungsindizes wenigstens einmal für jeden Schallstrahl sein Weg zur Errechnung seiner Laufzeit zumindest annähernd bestimmt wird und aus gemessener und errechneter Laufzeit der Ultraschallstrahlen Korrekturdaten zur schrittweisen Korrektur der Schallbrechungsindexverteilung erhalten werden, dadurch gekennzeichnet, daß zur Bestimmung des Weges eines Ultraschallstrahles die Integralgleichungen

berechnet werden, wobei d ein vorgegebener Faktor ist, der der Bedingung 0 < d < 1 genügt.

4. Anordnung zur Durchführung des Verfahrens nach einem der Ansprüche 1 bis 3 mit einer aus einer Mehrzahl von Sendeelementen bestehenden Ultraschallsendeanordnung und einer separaten, aus einer Mehrzahl von Empfängerelementen bestehenden Ultraschallempfängeranordnung, mit Mitteln zum Selektieren von Sende- und Empfangselementen derart, daß die jeweils aktivierten Sende- und Empfangselemente auf parallelen Verbindungsgeraden liegen mit Mitteln zum Messen der Laufzeit der Schallstrahlen zwischen dem jeweils aktivierten Sender- und Empfängerelementen und mit einer Rechenanordnung zur Bestimmung der Brechungsindexverteilung in einem zwischen Ultraschallsendeanordnung und der Ultraschallempfangsanordnung befindlichen Untersuchungsbereich, dadurch gekennzeichnet, daß die Rechenanordnung (29, 32) Mittel 321...323, 326...328) zur Berechnung des Abstandes (Y, Z) des Ultraschallstrahles (W'j) von der zugehörigen Verbindungsgeraden (Wj) an einer Vielzahl von Punkten nach einer Integralgleichung aufweist.

5. Anordnung nach Anspruch 4, dadurch gekennzeichnet, daß die Rechenanordnung Mittel zum Bestimmen der Brechungsindexverteilung und deren Gradienten in y- und z-Richtung auf den berechneten Ultraschallpfaden aufweist.

**Claims**

1. A method of determining the internal structure of a body where a plurality of ultrasonic beams traverse the body in different directions from each time one or more transmitter elements of an ultrasonic transmitter device in order to be converted into electric signals by one or more receiver elements of an ultrasonic receiver device, the transit times of the ultrasonic beams being measured, from the transit times and a preselected acoustic refraction index distribution which approximates the structure of the body there being determined at least once the approximate path of each sonic beam in order to calculate its transit time, correction data being derived from the measured and the calculated transit time of the ultrasonic beams for a step-wise correction of the acoustic refraction index distribution, characterized in that for the determination of the path of an ultrasonic beam the integral equations

$$Y = \int_{x0}^{x} \frac{w}{n} \int_{x0}^{s} gw \, dt \, ds + A \int_{x0}^{xF} \frac{w}{n} \, ds \qquad (1)$$

and

$$Z = \int_{x0}^{x} \frac{w}{n} \int_{x0}^{s} hw \, dt \, ds + B \int_{x0}^{x} \frac{w}{n} \, ds \qquad (2)$$

are solved, where x, t, s denote the direction of the straight connecting lines between the ultrasonic transmitter location x0 and the ultrasonic receiver location xf, Y and Z denote the distance between the path (W'j) and this straight line in two mutually perpendicular directions (y, z), n denotes the acoustic refraction index and g, h denote the gradients thereof in the y and the z-direction, respectively, w denotes the differential quotient of the path (W'j) with respect to x, and A and B are factors which are each time chosen so that the conditions Y(xf) = 0 and Z(xf) = 0 are satisfied.

2. A method as claimed in claim 1, characterized in that for the iterative determination of the path of a sonic beam each time the values n, g and h are determined for the coordinates (X, Y, Z) of the path (W'j) determined during a preceding cycle.

3. A method as claimed in one of claims 1 or 2, characterized in that from the newly calculated coordinates Y, Z and the coordinates Ya, Za applicable thus far for the same x value, the coordinates Yn, Zn of the new parth are calculated in accordance with the equations

Yn = dYa + (d–1)Y

Zn = dZa + (d–1)Z,

where d is a predetermined factor which satisfies the condition 0 < d < 1.

4. A device for performing the method claimed in any one of claims 1 to 3, comprising an ultrasonic transmitter device which consists of a plurality of transmitter elements, a separate ultrasonic receiver device which consists of a plurality of receiver elements, means for selecting transmitter elements and receiver elements in such a manner that the activated transmitter elements and receiver elements are always situated on parallel straight connecting lines, means for measuring the transit time of the sonic beams between the relevant activated transmitter elements and receiver elements, and an arithmetic device for determining the refraction index distribution in an examination zone situated between the ultrasonic transmitter device and the ultrasonic receiver device, characterized in that the arithmetic device (29, 32) includes means (321...323, 326...328) for calculating, using an integral equation, the distance (Y, Z) between the ultrasonic beam (W'j) and the associated straight connecting line (Wj) in a plurality of points.

5. A device as claimed in claim 4, characterized in that the arithmetic device comprises means for determining the refraction index distribution and its gradients in the y and the z-direction on the calculated ultrasonic paths.

**Revendications**

1. Procédé pour déterminer la structure interne d'un corps consistant à faire traverser le corps dans différentes directions par une pluralité de faisceaux ultrasonores émis par un ou plusieurs éléments émetteurs d'un dispositif émetteur d'ultrasons pour les convertir en signaux électriques par un ou plusieurs éléments récepteurs d'un dispositif récepteur d'ultrasons, à mesurer les temps de transit des faisceaux ultrasonores et, à partir des temps de transit et d'une répartition d'indice de réfraction acoustique prédéterminée et se rapprochant de la structure du corps, à déterminer au moins approximativement et au moins une fois pour chaque faisceau ultrasonore le trajet de ce faisceau ultrasonore pour le calcul de son temps de transit et à déduire du temps de transit mesuré et calculé des faisceaux ultrasonores des données de correction pour la correction par étapes de la répartition de l'indice de réfraction acoustique, caractérisé en ce que, pour déterminer le trajet d'un faisceau ultrasonore, sont résolues les équations intégrales

$$Y = \int_{x0}^{x} \frac{w}{n} \int_{x0}^{s} gw \ dt \ ds + A \int_{x0}^{x} \frac{w}{n} \ ds \qquad (1)$$

et

$$Z = \int_{x0}^{x} \frac{w}{n} \int_{x0}^{s} hw \ dt \ ds + B \int_{x0}^{x} \frac{w}{n} \ ds \qquad (2)$$

où x et t, s indiquent la direction de la ligne droite de jonction entre l'endroit d'émission d'ultrasons x0 et l'endroit de réception d'ultrasons xf, Y et Z indiquent la distance qui sépare le trajet (W'j) de cette ligne droite dans deux directions perpendiculaires (y, z), n représente l'indice de réfraction acoustique et g et h le gradient de celui-ci dans la direction y d'une part et z d'autre part et w est le quotient différentiel du trajet (W'j) suivant x et A et B sont des facteurs choisis de façon qu'on satisfasse aux conditions Y(xf) = 0 et Z(xf) = 0.

2. Procédé selon la revendication 1, caractérisé en ce que pour la détermination itérative du trajet d'un faisceau ultrasonore, sont déterminées chaque fois les valeurs n, g et h pour les coordoonnées (X, Y, Z) du trajet (W'j) déterminé dans un cycle précédent.

3. Procédé selon l'une des revendications 1 et 2, caractérisé en ce qu'à partir des coordonnées Y, Z nouvellement calculées et des coordonnées Ya, Za jusqu'ici valables pour la même valeur x, sont calculées les coordonnées Yn, Zn du nouveau trajet selon les relations

Yn = dYa + (d−1)Y

Zn = dZa + (d−1)Z

où d est un facteur prédéterminé satisfaisant à la condition 0 < d < 1.

4. Dispositif de mise en œuvre du procédé selon l'une des revendications 1 à 3, comportant un dispositif émetteur d'ultrasons constitué d'une pluralité d'éléments émetteurs et un dispositif récepteur d'ultrasons séparé constitué d'une pluralité d'éléments récepteurs, des moyens pour sélectionner des éléments émetteurs et récepteurs de telle manière que les éléments émetteurs et récepteurs activés soient situés sur des lignes droites de jonction parallèles, des moyens pour mesurer le temps de transit des faisceaux ultrasonores entre les éléments émetteurs et récepteurs activés, et un dispositif de calcul pour déterminer la répartition d'indice de réfraction dans une zone d'examen située entre le dispositif émetteur d'ultrasons et le dispositif récepteur d'ultrasons, caractérisé en ce que le dispositif de calcul (29, 32) comporte des moyens (321...323, 326...328) pour calculer à une pluralité de points et au moyen d'une équation intégrale la distance (Y, Z) qui sépare le faisceau (W'j) de la ligne droite de jonction correspondante (Wj).

5. Dispositif selon la revendication 4, caractérisé en ce que le dispositif de calcul comporte des moyens pour déterminer la répartition d'indice de réfraction et son gradient dans une direction y et z dans les trajets calculés de faisceau ultrasonore.

FIG.1

FIG.7

$(n,g,h)$ $W_j$ — 35

$J = \int_{x0}^{S} g\ dt$ — 40

$K = \int_{x0}^{X} J/n\ ds$ — 50

$L = \int_{x0}^{X} \frac{1}{n}\ ds$ — 60

$Y =$ — 70

$Y_n = d \cdot Y_a + (1-d)\ Y$ — 36

$(n,g,h)$ $W_j'$ — 37

**FIG.2**

$K(0) = l1 \cdot J(1) / 2\ n(1)$ — 51

For $i = 1, \cdots, N$ — 52

$K(i) = K(i-1) + \frac{05}{n(i)} (J(i) + J(i-1))$ — 53

$K(N+1) := K(N) + (K(N) + J(N)) \cdot l2 / 2\ n(N)$ — 54

50

**FIG.4**

40

$J(0) = g(1) \cdot l1$ — 41

For $i = 1, \cdots, N$ — 42

$J(i) = g(i) + J(i-1)$ — 43

$J(N+1) := J(N) + g(N) \cdot l2$ — 44

**FIG.3**

**FIG.5**

60

$L(0) = l1 / n(1)$ — 61

For $i = 1, \cdots N$ — 62

$L(i) = \frac{1}{n(i)} + L(i-1)$ — 63

$L(N+1) := L(N) + l2 / n(N)$ — 64

$A = -K(N+1) / L(N+1)$ — 71

For $i = 1, \cdots N$ — 72

$Y(i) = K(i) + A \cdot L(i)$ — 73

70

**FIG.6**